# EUROPEAN PATENT APPLICATION

(11) **EP 1 449 814 A1**
(43) Date of publication of application: **25.08.2004**
(21) Application number: 04003021.5
(22) Date of filing: 11.02.2004
(51) Int. Cl.: C02F 3/34, C12N 1/14, C12N 1/20

(54) **Fungi and their symbiotic bacterial group suitable for treating organic waste, and uses thereof**

(30) Priority: 21.02.2003 JP 2003044321
(71) Applicant: Sadaie, Tamiko, Mishima-city, Shizuoka-ken (JP); Homer Clean Corporation, Tokyo (JP); Wakao, Kichi, Kyoma-gun, Yamanashi-ken (JP)
(72) Inventor: Sadaie, Tamiko, Mishima-City Shizuoka-ken (JP)
(74) Representative: Heusler, Wolfgang, Dipl.-Ing.

(57) **Abstract**

Provided are fungi and their symbiotic bacterial group suitable for decomposing/purifying organic waste and deodorizing a fetid source. The fungi and their symbiotic bacterial group are symbiotic flora which grow together in an environment where the oxygen concentration is kept essentially at 1 ppm or less, by metabolizing carbon sources utilizing inorganic salts as an electron-acceptor, and comprise, as predominant organisms, following microbes: Mucor indicus, Myxococcus sp., Flavobacterium johnsoniae, Pseudomonas alcaligenes, Klebsiella ornitinolytica, Bacillus licheniformis, Bosea thiooxidans, and Methylosinus tricosporium.

## Description

### BACKGROUND OF THE INVENTION

### FIELD OF THE INVENTION

The present invention relates to fungi and their symbiotic bacterial group for treating organic waste by decomposing, purifying and/or deodorizing it. The invention further relates to an effective uses thereof.

### DETAILED DESCRIPTION OF PRIOR ART

Many studies have been carried out in order to develop methods enabling the effective disposal of pollutants and waste materials which, unless disposed of properly, would have a grave adverse effect on the environment. Among them, biological waste disposal methods using microbes rapidly attract attention in recent years partly because they are free from the risk of invoking secondary environmental pollution in contrast with chemical waste disposal methods, and partly because according to those methods disposal of pollutants and waste materials proceeds as a natural process occurring in an ecological system consisting of symbiotic microbes.

Treatment of organic waste using microbes has long been employed as a common means for purifying liquid waste. However, no sufficient studies have yet been made as to the proper ecological system of microbes which grow by digesting organic waste, thereby purifying the waste. Indeed, bad smells and sludge residue which are generated during and subsequent to biological treatment of liquid waste, and considered to be a problem associated with the biological treatment have been resolved by adding two separate units to the sewage cleaning system, one for deodorizing the waste during its biological treatment and the other for further treating the remaining sludge in a separate tank.

Some studies have been published as to the microorganisms useful for decomposing or deodorizing organic waste. Aerobic bacterial species cited in those studies to be used for aerobic treatment include, for example, Zooglea, Achromobacter, Alcaligenes, Bacillus, Pseudomonas, etc. Anaerobic bacterial species to be used for anaerobic treatment include, for example, Desulfovibrio, Methanomonas, etc. Bacterial species to be used for decomposing odorous materials include, for example, Nitrobacter which decomposes ammonia, Chlorobium which decomposes sulfur-containing compounds, and Cl-compound assimilating bacteria such as those belonging to Genera Hyphomicrobium and Thiobacillus (Toshio OMORI, "Environmental Biotechnology" 2001, published in Japan).

However, ordinary aerobic treatment results in the production of a great amount of residual sludge. Moreover, in order to allow a system of deodorizing microbes as described above to completely decompose, by oxidation, ammonia and sulfur-containing compounds contained in waste into odorless, inorganic elements, it is necessary to exactly adjust the amount of oxygen supplied to the deodorizing microbe group. The exact control of oxygen supply to a microbe group is so difficult that it is practically impossible to completely eliminate bad smells from waste using such a system. In addition, since ordinary anaerobic treatment consists of confining microbes together with waste in an anaerobic environment so that the microbes can digest the waste, the problem of producing a rich amount of odorous materials after treatment remains unsolved.

Some patents propose the adoption of bacterial species including new ones for treating or deodorizing organic waste. For example, the Japanese Patent Application Publication No. 2001-224365 proposes a microorganism-containing compound useful for eliminating slurry adherent to the toilet stool or kitchen sink, and its foul odor, which is obtained by adding sodium hydrogencarbonate, glucose and alum to microorganisms belonging to Genus Bacillus capable of producing amylase, protease and lipase. Further, Japanese PCT Patent Application Publication No. 2002-528113 discloses an invention in which microbes are separated from soil; among them those that are effective for treating sewage are identified (four Actinomyces species, and one belonging to Genus Bacillus); the microbes are used for treating and deodorizing sewage discharged from livestock pens; and the supernatant of treated sewage is used as a deodorizing agent or liquid fertilizer.

However, the majority of the microbes used both in the Japanese Patent Application Publication No. 2001-224365 and Japanese PCT Patent Application Publication No. 2002-528113 employ oxygen as an electron-acceptor, and thus to sustain their growth it is necessary to supply a huge amount of oxygen. Thus, the purification and deodorizing systems proposed in those patents share the same problems encountered with the above aerobic treatment. The Japanese Patent Application Publication No. 2001-224365 further discloses a method for accelerating the decomposition of organic waste, by adding thereto microbes appropriate for the kind of given organic waste. Therefore, it is necessary to sequentially add a series of microbe groups to organic waste during the course of its decomposition until the organic waste is completely decomposed. With regard to the bacterial species disclosed in the Japanese PCT Patent Application Publication No. 2002-528113, their decomposing and deodorizing activities are tested only on sewage from livestock pens, and feasibility of producing a fertilizer from decomposed and deodorized sewage is tested only on the same sewage. Namely, the invention in question does not mention at all as to what effects those microbes have in the treatment and odor-elimination of organic waste at large.

### SUMMARY OF THE INVENTION

The present invention is to provide a method which comprises using a group of microbes (fungi and their symbiotic bacterial group) which are distinct from the species of microbes used in usual sewage purification systems, for decomposing and purifying organic waste, and deodorizing it by decomposing odorous materials. The fungi and their symbiotic bacterial group provided by the invention (microbe group of the invention) can digest organic waste which serves as a carbon source using inorganic salts as an electron-acceptor in an environment where the level of oxygen content is kept essentially at 1 ppm or less. In the concrete, the microbe group of the invention includes, to mention predominant ones, following organisms:
Mucor indicus (ATCC90364),
Myxococcus sp. (ATCC49305),
Flavobacterium johnsoniae (ATCC23107),
Pseudomonas alcaligenes (ATCC14909),
Klebsiella ornitinolytica (ATCC31898),
Bacillus licheniformis (ATCC14580),
Bosea thiooxidans (ATCC700366),
Methylosinus tricosporium (ATCC35070).

The aforementioned inorganic salt includes at least nitrate. The aforementioned carbon source is organic material containing cellulose compounds.

The present invention provides an agent comprising the aforementioned fungi and their symbiotic bacterial group for treating organic waste, and an agent for deodorizing organic waste.

Further, the present invention provides a method for treating organic waste which comprises adding the aforementioned fungi and their symbiotic bacterial group to organic waste for mixture, and allowing that microbe group to decompose and purify the organic waste.

And further, the present invention provides a method for deodorizing organic waste which comprises adding the aforementioned fungi and their symbiotic bacterial group to the organic waste and allowing that microbe group to deodorize the organic waste by decomposing odorous materials.

### DESCRIPTION OF PREFERRED EMBODIMENTS

The inventor of the present invention had long studied from a bacteriological viewpoint to seek a method for effectively treating liquid waste, and found that fungi and their symbiotic bacterial group appearing in sewage under certain conditions are quite effective not only for decomposing and purifying a wide variety of organic wastes, but also for deodorizing those wastes, and reached this invention. The fungi and their symbiotic bacterial group can grow cooperatively in the presence of a carbon source and an electron-accepter, in an environment where the level of oxygen is kept essentially at 1 ppm or less.

The fungi and their symbiotic bacterial group provided by the invention are effective not only for decomposing and purifying organic waste such as sewage from factories, sewage from common household, sewage from toilet, raw trash, fresh waste from toilet and latrine, plant waste or the like, but also for deodorizing such organic waste.

According to the invention, the allowable limit of oxygen concentration is essentially 1 ppm (1 mg/L) or lower. This is because, if the level of oxygen were above the aforementioned limit, aerobic species would be more activated which would lead to the conventional type of waste treatment based on the activation of sludge. The term "essentially" is used here to mean that the upper limit of oxygen concentration may fall around 1 ppm including a minute range above that limit over which the fungi and symbiotic bacterial group of the invention can safely grow. It is possible to supply the above level of oxygen to the microbe group of the invention, as follows. When purification of liquid waste is required, the amount of oxygen dissolved in the waste after the aeration treatment is adjusted properly. When the odor of waste from livestock pens must be eliminated, exposure of the waste to atmosphere is adjusted by, for example, covering the waste with a vinyl sheet.

In an environment where the level of oxygen is adjusted to the above level, the microbe group comprising the fungi and their symbiotic bacterial group of the invention respire using, as an electron-acceptor, oxygen which serves as an easily accessible energy source, and grow using organic materials as a nutritional source. When oxygen is used up (i.e., dissolved oxygen becomes exhausted, or the level of oxygen becomes zero), the microbe group respire using inorganic salts or another chemical constituent of the waste as an electron-acceptor. The inorganic salts include at least nitrate. In addition, they may include sulfate (containing thiosulfate), iron ingredient, manganese ingredient, fumarate, etc. Of those inorganic salts, at first nitrate is consumed according to its oxidation-reduction potential. Then, the other salts are also consumed being used as electron-acceptors (solution containing such inorganic salts will be called a controlled electron-acceptor solution hereinafter).

The electron-acceptor solution may contain, for example, nitrates at 6 ppm, sulfates at 12 ppm, and thiosulfates at 1 ppm. However, the contents of those inorganic salts in the controlled electron-acceptor solution are not limited to any specific ranges but may vary depending on the environment where an involved microbe group of the invention grows. Once the aforementioned inorganic salts which serve as electron-acceptors are added to waste to be treated, they will be then produced by the microbe group itself existent in the waste, and thus no additional supply of those salts will be necessary as long as the microbe group stably grow on the waste. The carbon source is a nutrient upon which the microbe group grows, and consists of organic materials comprising cellulose compounds such as cellulose, hemicellulose, and the like.

The microorganisms appearing in the above described environment were isolated, and the base sequence of DNA of each isolate was determined for identifying the isolate. As a consequence it was found that the microbe group of the invention predominantly comprises fungi accompanied with symbiotic bacteria as specified below:
1. Mucor indicus (ATCC90364);
2. Myxococcus sp. (ATCC49305);
3. Flavobacterium johnsoniae (ATCC23107);
4. Pseudomonas alcaligenes (ATCC14909);
5. Klebsiella ornitinolytica (ATCC31898);
6. Bacillus licheniformis (ATCC14580);
7. Bosea thiooxidans (ATCC700366); and
8. Methylosinus tricosporium (ATCC35070).

ATCC cited above is an abbreviation of the American Type Culture Collection, and those microbes cited above are readily available from this organization.

The microbe group (the fungi and their symbiotic bacterial group) is obtained by transferring sewage containing organic materials into an aeration tank, aerating the sewage in such a manner as to allow the concentration of oxygen dissolved in the sewage to be 1 ppm or less, and extracting the supernatant. More preferably, the microbe group is obtained by separating (depositing) a sediment from the above aerating sewage liquid , aerating again the sediment in such a manner as to allow the concentration of oxygen dissolved in the sediment to be 1 ppm or less, and extracting the supernatant.

Individual microbes cited above are known. However, the microbe group of the invention where individual microbes are in symbiotic relations with each other in terms of catabolism is capable of decomposing organic materials, and decomposing odorous metabolites.

The microbe group of the invention where individual microbes are in symbiotic relations with each other in terms of catabolism grow on organic materials, so it is considered, via a sort of cascade processes: at an initial phase of catabolism certain organic materials are digested by one species of microbes into intermediates which are then digested by another species of microbes into further decomposed intermediates, and the process is repeated until the initial organic materials are reduced to basic inorganic elements. These cascade processes result in decomposition of odorous intermediates during this catabolic process. The fungi and their symbiotic bacterial group are basically weakly aerobic, and grow using, as a carbon and energy source, protein metabolites such as oligopeptides, amino acids, organic acids, etc. Or, they digest ammonia and hydrogen sulfate which are left by certain other organisms as end products, or the oxides of those compounds to gain energy therefrom. However, since the microbe group in question is mixed with other microbes growing on organic matters, finally the system digests organic matters in collaboration with other microbes which are also sustainable under the aforementioned condition.

The microbes identified by numbers 1, 2, 3 and 6 above (initially active group) secrete mucous fluid which contains amylase, protease, nuclease, lipase and cellulase which, when brought into contact with organic matter, digest it and leave by-products. The by-products attract another group of organisms including microbes 4 and 5 mentioned above (mid-term active group). The by-products are then decomposed further into inorganic elements which may be digested by a third group of microbes 7 and 8 (finally active group). For the most part, the mucous secret is composed of proteins. It is thought that the mid-term active group, when they consume the by-products or external supply of nutrients, will digest the proteins contained in the secret to maintain their life.

The aforementioned fungi and their symbiotic bacterial group can be used in the production not only of a treatment agent for decomposing/ purifying organic waste but also of a deodorizing agent for deodorizing such organic waste. The treatment agent and/or deodorizing agent described above may be prepared by subjecting sewage to a renewed aeration in such a manner as to allow its oxygen content to be 1 ppm or less, extracting the supernatant (in a liquid) therefrom, applying the supernatant to a cellulose substrate consisting, for example, of rice-bran, saw dusts or straws which serves as a culture bed, to thereby inoculate the fungi and their symbiotic bacterial group to the culture bed, incubating the culture under a weakly aerobic condition (oxygen conc. being 1 ppm or less), and drying the resulting culture and pulverizing the solid into a powder using conventional methods.

When the treatment agent for organic waste prepared as above is applied to organic waste such as sewage, trash, fresh discharge from toilets and latrines or the like in an environment where the oxygen concentration is kept at 1 ppm or less, it is possible to decompose organic matters contained in the waste, to thereby purify the waste. Treatment of sewage consists of adding the treatment agent for organic waste to raw liquid waste, aerating the liquid waste such that the level of dissolved oxygen (DO) is kept essentially at 1 mg/L or less, allowing precipitates contained in the liquid waste to settle to form a sediment or sludge, separating the sludge from the supernatant which is treated conventionally, subjecting the sludge to a renewed aeration such that the level of dissolved oxygen (DO) is kept essentially at 1 mg/L or less, separating the supernatant from the sludge which is treated conventionally, and preparing a treatment agent from the supernatant or transferring the supernatant to raw liquid waste to use it as a treatment agent.

The deodorizing agent, when applied to organic matter emitting a foul odor, eliminates the foul odor by decomposing odorous constituents of the organic matter. This is in contrast with conventional deodorizing agents mainly comprising bacteria which are specialized in digesting fetid substances such as sulfates, methane gas, ammonia and the like. Namely, the deodorizing agent of the invention depends on the coordinated activity of a microbe group comprised mainly of fungi and their symbiotic bacteria group which can respire using oxygen and inorganic salts as their electron acceptors, and thus smoothly metabolize organic matter while scarcely producing malodorous intermediates during the course of metabolic activity.

Examples representing the invention will be described below. It should be understood, however, that the scope of the invention is not limited in any way to those examples.

### (Examples)

Domestic sewage was aerated in an experimental tank in such a manner as to allow the level of dissolved oxygen to be kept at 1 ppm or less. Flora contained in the supernatant were sampled. They were placed in a medium, stirred and suspended. Then, they were diluted to an appropriate concentration, incubated on an LB medium, and separated into individual species for identification. The fungi were distinguished depending on the base sequence of ribosomal 18S RNA, while the bacteria based on the corresponding sequence of ribosomal 16S RNA. Myxococci were identified by microscopy. Properties of the organisms thus isolated and identified are listed in Table 1.

**Table 1**

| Name of organisms | Electron acceptor | Electron donor | Excreted enzymes | Note |
|---|---|---|---|---|
| Mucor indicus | Oxygen | Sugar, organic acid | | |
| Flavobacterium johnsoniae | Oxygen, nitrate | Cellulose | Cellulase | Denitrification |
| Pseudomonas alcaligenes | Oxygen, nitrate | Organic acid, amino acid | | |
| Klebsiella ornitionolytica | Fumarate (fermentation) | Organic acid | | Nitrification |
| Bacillus licheniformis | Oxygen, nitrate | sugar | Protease, cellulase, etc. | Denitrification |
| Bosea thiooxidans | Oxygen | Organic acid, amino acid | | Oxidation of sulfides |
| Methylosinus tricosporium | Oxygen (weakly aerobic) | Carbon compound, hydrogen | | Deodorizing, Assimilation of C₁ compounds |
| Mixococcus sp. | Oxygen | Oligopeptide | Protease, lipase, etc. | Secretion of protein-rich mucus, & antibiotic substances |

The fungi and their symbiotic bacterial group and controlled electron-acceptor solution were prepared as described above. They were transferred into experimental tanks containing domestic sewage, sewage from kitchen containing minced trash (kitchen sewage), sewage from pig pens (pig pen sewage), and sewage from food processing plants (food plant sewage) . Each tank content was aerated in such a manner as to allow the content of dissolved oxygen to be 1 ppm or less. A sample was extracted from the supernatant of each tank content, and its physico-chemical properties were determined to evaluate the quality of treated water.

**Table 2**

| (Treatment of domestic sewage) | | | | | | | |
|---|---|---|---|---|---|---|---|
| | pH | BOD*1 | COD*2 | SS*3 | N-hexane extraction | Total nitrogen | Total phosphor |
| Sewage | 7.1 | 17 | 14 | 22 | 0.5> | 22.7 | 1.1 |
| Treated water | 6.4 | 5.7 | 3.3 | 6.2 | 0.5> | 8.8 | 1.4 |

| | | | | | | | |
|---|---|---|---|---|---|---|---|
| *1: Biological oxygen demand | | | | | | | |
| *2: Chemical oxygen demand | | | | | | | |
| *3: Suspended solid | | | | | | | |

**Table 3**

| (Treatment of kitchen sewage) | | | | | | |
|---|---|---|---|---|---|---|
| | pH | BOD | SS | N-hexane extraction | Total nitrogen | Total phosphor |
| Sewage | 6.9 | 1886 | 1069 | 347 | 75 | 16 |
| Treated water | 7.6 | 105 | 116 | 6.2 | 22.6 | 9.3 |

**Table 4**

| (Treatment of pig pen sewage) | | | | | | |
|---|---|---|---|---|---|---|
| | pH | BOD | COD | SS | Total nitrogen | Total phosphor |
| Sewage | 6.8 | 5100 | 1600 | 2700 | 560 | 100 |
| Treated water | 8.1 | 70 | 430 | 76 | 150 | 25 |
| Residual E. coli count: 1.3 x 10² | | | | | | |

**Table 5**

| (Treatment of food plant sewage) | | | | | | | |
|---|---|---|---|---|---|---|---|
| | pH | BOD | COD | SS | N-hexane extraction | Total nitrogen | Total phosphor |
| Sewage | 6.3 | 1100 | 360 | 230 | 11 | 10.6 | 3.9 |
| Treated water | 7.6 | 28 | 34 | 19 | 0.5> | 8.07 | 0.84 |

As shown in the tables, for all the sewage samples tested, the total contents of nitrogen- and phosphor-containing compounds were greatly reduced, suggesting the marked improvement of quality of the test sewage. The odor of the samples was eliminated after treatment, and their residual sludge was also greatly reduced. A microbe group of the invention suspended in a controlled electron-acceptor solution was mixed with a compost sample (consisting of raw trash and livestock manure) in an environment where the oxygen level is kept equal to 1 ppm or less. Then, it was found that the compost sample was decomposed highly effectively without emitting any notable odor.

Controlled electron-acceptor solutions each containing a microbe group of the invention were added to various fetid samples to evaluate the deodorizing activity of the microbe group. The results are shown in the table below.

**Table 6**

| Fetid matter | Odor classification | Deodorizing activity | Application method |
|---|---|---|---|
| Domestic sewage tank | Sewage odor | +++ | Mixing |
| Pig manure | Strong ammoniac odor | ++ | Spraying |
| Fowl manure | Fowl manure odor | ++ | Spraying |
| Cattle manure | Fermented organic acid | ++ | Spraying |
| Raw trash | Acidic odor | ++ | Spraying |
| Pen, pet house | Ammoniac odor | +++ | Spraying |

As is obvious from inspection of the table, the microbe group of the invention exerted a marked deodorizing effect on all the fetid samples, suggesting the excellent deodorizing activity of the microbe group. The deodorizing activity of the test solution towards a fetid sample was quantified by the number of plus symbol (+) : the higher the deodorizing activity of the test solution is to a given fetid sample, the more the number of plus symbol is attached to the sample.

As discussed above, the fungi and their symbiotic bacterial group of the invention can decompose/purify organic waste while decomposing foul odor from the waste during treatment, and thus the microbe group of the invention can be suitably used as a treatment and deodorizing agent for organic waste. Moreover, if the fungi and their symbiotic bacterial group of the invention are combined with a controlled electron-acceptor solution, and then the suspension is applied to organic waste, the microbe group will produce electron-acceptors by themselves as a result of their physiological action on the waste, which in turn accelerates their overall growth, thus obviating the need for additional supply of the microbe group, and simplifying works involved in the management of the sewage purification system.

## Claims

1. Fungi and their symbiotic bacterial group suitable for treating organic waste, **characterized in that** the fungi and their symbiotic bacterial group being produced by;
growing together in an environment where the oxygen concentration is kept essentially at 1 ppm or less, with carbon sources for a nutrient and electron-accepters including inorganic salts.

2. The fungi and their symbiotic bacterial group as described in Claim 1, the fungi and their symbiotic bacterial group, as predominant organisms, comprising;
Mucor indicus (ATCC90364),
Myxococcus sp.(ATCC49305),
Flavobacterium johnsoniae (ATCC23107),
Pseudomonas alcaligenes (ATCC14909),
Klebsiella ornitinolytica (ATCC31898),
Bacillus licheniformis (ATCC14580),
Bosea thiooxidans (ATCC700366), and
Methylosinus tricosporium (ATCC35070).

3. The fungi and their symbiotic bacterial group as described in Claim 1, in which the inorganic salts as electron-acceptors include at least nitrates.

4. The fungi and their symbiotic bacterial group as described in Claim 1, in which the carbon sources are organic matter including cellulose substances.

5. An agent for treating organic waste, **characterized in that**;
fungi and their symbiotic bacterial group produced by growing together in an environment where the oxygen concentration is kept essentially at 1 ppm or less, with carbon sources for a nutrient and electron-accepters including inorganic salts.

6. A method for treating organic waste, **characterized in that**;
mixing fungi and their symbiotic bacterial group with the organic waste, and decomposing the organic waste, in which the fungi and their symbiotic bacterial group produced by growing together in an environment where the oxygen concentration is kept essentially at 1 ppm or less, with carbon sources for a nutrient and electron-accepters including inorganic salts.

7. An agent suitable for deodorizing organic waste, **characterized in that**;
fungi and their symbiotic bacterial group produced by growing together in an environment where the oxygen concentration is kept essentially at 1 ppm or less, with carbon sources for a nutrient and electron-accepters including inorganic salts.

8. A method for deodorizing a fetid source containing organic matter, **characterized in that**;
mixing fungi and their symbiotic bacterial group with the fetid source, and decomposing odorous materials, in which the fungi and their symbiotic bacterial group produced by growing together in an environment where the oxygen concentration is kept essentially at 1 ppm or less, with carbon sources for a nutrient and electron-accepters including inorganic salts.
